# EUROPEAN PATENT SPECIFICATION

(11) **EP 0 449 960 B1**
(45) Date of publication and mention of the grant of the patent: **08.09.1993**
(21) Application number: 90901403.7
(22) Date of filing: 15.12.1989
(51) Int. Cl.: A61K 9/107, A61K 9/14, A23D 7/00

(54) **HEAT-DEHYDRATED EMULSION COMPOSITIONS**
DURCH HITZE DEHYDRATISIERTE EMULSIONEN
COMPOSITIONS D'EMULSIONS DESHYDRATEES A LA CHALEUR

(30) Priority: 20.12.1988 US 287127; 20.09.1989 US 409822
(43) Date of publication of application: 09.10.1991
(73) Proprietor: MEDICONTROL CORPORATION, Newton MA 02164 (US)
(72) Inventor: TABIBI, Esmail, Chelmsford, MA 01824 (US); SICILIANO, Arthur, A., Framingham, MA 01701 (US)
(74) Representative: Dubois-Chabert, Guy
(86) International application number: US8905632
(87) International publication number: WO9006746

(56) References cited:
- EP-A- 0 211 257
- EP-A- 0 276 735
- CH-A- 414 950
- FR-A- 2 494 992
- US-A- 3 514 298

## Description

This invention relates generally to the preparation and composition of dehydrated (dried) oil-in-water emulsions. More specifically, it relates to compositions dried at room temperature or above and intended for oral or parenteral use as therapeutics, nutrients, or biologics which are easily reconstituted with water at the time of administration to humans or animals. The compositions and methods of preparation described herein offer substantial advantages over other methods of preparing dehydrated emulsions for reconstitution.

Numerous compositions for oral or parenteral administration are desirable in the form of oil-in-water emulsions. These include oral (enteral) feeding systems of edible vegetable fats or oils, emulsified using approved emulsifiers, which may also contain other nutrients such as amino acids, vitamins and sugars in the aqueous phase. Similar preparations based on metabolizable vegetable oils, biocompatible emulsifiers, soluble nutrients, buffers and water are useful as concentrated calorie sources in parenteral nutrition. Likewise, such parenteral emulsions of submicron size are used to deliver water-insoluble, lipid-soluble drugs. Enteral emulsions can also be used to deliver such drugs orally. Such emulsions have potential advantages over other delivery forms which include side effect reduction, controlled or sustained release, higher activity, improved targeting, enhanced biocompatibility, increased stability, improved compatibility with other dosage forms, and improved economics.

Oil-in-water liquid emulsions for oral and parenteral use have drawbacks which make their preparation, transportation, storage, and administration inconvenient. Liquid emulsions containing water cannot be employed with drugs which will degrade in the presence of water. Also, the bulk and overall weight of the aqueous phase often makes transportation uneconomic. Liquid emulsions often have limited shelf life stability due to the tendency of the immiscible phase to separate with time. Concentrations of drugs per unit of fluid are fixed in liquid emulsions. Also, liquid emulsions provide fertile growth media for microorganisms and are difficult both to preserve and to sterilize.

One attempt at dehydrating emulsions similar to those of the present invention is disclosed in European Publication 0,211,257 of Abbott Laboratories, published February 25, 1987. This publication requires the use of a complex freeze-drying process to dehydrate the emulsion, and then a multi-day period in a lyophilizer slowly increasing the temperature. Even after this extensive and expensive period, reconstitution by the addition of water produces particles which are substantially larger than those that were in the original emulsion.

Non-protein high stability fat emulsion compositions were prepared in U.S. Pat. No. 4,492,714 (Cooper et al.). The compositions contain 10 to 75% edible fat, 0.1 to 3% emulsifier, 8 to 20% starch, and the remainder filler. They are intended to be dried to low moisture contents and then rehydrated during use, i.e. as a substitute for cream in coffee. Preparing the Cooper et al. compositions requires, as the critical component, that the starch be a hydrated undextrinized lipophilic starch, predominantly in submicroscopic particle size. As shown by the comparative examples below, the substitution of such a starch for the hydrophilic water-soluble carbohydrate component of the present invention does not produce a rehydratable emulsion.

European Apppln. 276,735 of BASF discloses the use of organic solvents to prepare aqueous and dehydrated pharmaceutical preparations containing a protective colloid which is generally water-insoluble. The preparations also contain a therapeutic and lipophilic agent, an edible oil or fat, and an emulsifier. The present emulsions are completely liquid and avoid the use of organic solvents in their preparation.

It is an object of the present invention to produce a dehydrated emulsion composition which when reconstituted will yield droplets which are essentially of the same size as those that were in the emulsion prior to the dehydration. It is a further object of this invention to produce such dehydrated emulsions without the use of a freeze-drying procedure and consequent three-day lyophilization. It is a still further object to produce dehydrated emulsions which will be storage stable for extended periods, easily sterilized, reconstitutable to a variety of concentrations, easily transported, easily preserved, and useful for the delivery of lipid-soluble materials, e.g. therapeutics, biologicals, and nutrients, to humans and animals. It is a still further object to provide a method of preparing such reconstitutable dehydrated oil-in-water emulsions.

It has been discovered that an oil-in-water emulsion having a continuous aqueous phase containing therein discontinuous oil phase droplets of less than about 0.4 microns average diameter may be dehydrated at a temperature of at least about 20°C. and subsequently reconstituted by the addition of water to produce an oil-in-water emulsion wherein the oil phase droplets again have an average diameter of less than about 0.4 µm. The initial emulsion is prepared in the absence of any organic solvent and from a combination of 0.1 to 60 weight percent of a lipid-soluble material, 0.1 to 10 weight percent of a oil-in-water emulsifier, and 0.5 to 70 weight percent of a hydrophilic water-soluble solid carbohydrate, plus 20 to 99 weight percent of water.

The dehydrated emulsions of the present invention are formed by dehydrating a precursor oil-in-water emulsion containing all of the desired ingredients. The dehydration is performed at a temperature of about room temperature, i.e. at least about 20° C., or above. The resultant dry product contains a minimal amount of moisture and may be reconstituted by the addition of water to produce an oil-in-water emulsion having essentially identical pharmaceutical activity as the original emulsion before dehydration, as shown by the oil phase droplets having essentially the same size as in the original emulsion. The oil phase droplets in both cases exhibit average diameters less than about 0.4, preferably less than about 0.3, µm. The dehydration and rehydration procedures may cause the oil phase droplets to increase in average diameter, but they still remain smaller than about 0.4 µm and thus essentially completely equivalent in performance to the original emulsion. Normally the increase in average diameter is less than about 50, preferably less than about 25, most preferably less than about 10, percent.

The precursor oil-in-water emulsion is prepared from a mixture of a lipid-soluble material, an oil-in-water emulsifier, a hydrophilic, water-soluble carbohydrate which is solid at room temperature, and water. Generally the mixture will comprise 0.1 to 60 weight % lipid-soluble material, 0.1 to 10 weight % emulsifier, 0.5 to 70 weight % carbohydrate, and 20 to 99 weight % water. More preferably, the initial emulsion is prepared from 1 to 50 weight % lipid-soluble material, 0.5 to 5 weight % emulsifier, 5 to 50 weight % carbohydrate, and 30 to 95 weight % water. Most preferably, the initial emulsion is prepared from 5 to 20 weight % lipid-soluble material, 1 to 3 weight % emulsifier, 5 to 40 weight % carbohydrate, and 40 to 95 weight % water. The emulsions may also contain such conventional additives as buffers, stabilizers/-preservatives, flavors or colors in conventional amounts for pharmaceutical compositions.

Any lipid-soluble material may be used in the present invention. Preferably, the lipid-soluble material will be selected based upon the dehydration temperature so that it is not degraded by the heat used to dehydrate the emulsion. Examples of suitable lipid-soluble materials include lipids per se, nutrients, therapeutics, and biologics. Examples of lipids useful herein include foods, vegetable and fish oils, triglycerides, synthetic or semi synthetic mono or di, triglycerides. Lipid-soluble nutrients or drugs useful herein include those active agents which are relatively water-insoluble but are quite oil-soluble. In certain cases, the lipophilic active can also represent the lipid component.

Lipid-soluble (lipophilic and amphiphilic) therapeutic and biological agents useful herein are preferably selected from the group consisting of adrenal cortical steroids, adrenal cortical steroid inhibitors, allergens, analeptic agents, analgesics, anesthetics, anorexics, antialcohol preparations, anti-arthritics, antiasthma, antibacterials, antiseptics, antibiotics, anticatecholamine synthesis, anticholelithics, anticholinergics, inhibitors, anticoagulant antagonist, anticoagulant, anticonvulsants, antidepressants, antidiabetics, antidiarrheals, antidiuretics, antidotes, antienuresis, antifibrinolytics, antifibrotics, antiflatulents, antifungals, antigonadotropins, antiherpes, anti-aids, antihistamines, antihyperammonia, antiinflamratories, antileprosy, antimetabolites, antimigraines, antimotion sickness, antinauseants, antineoplastics, antiparasitics, antiparkinsonism, antiplatelet, antiporphyria, antipruritics, antipsychotics, antipyretics, antishock, antispasmodics, antivertigo, antiviral, vaccine antigens, globulins, toxoids, bone metabolism regulators, bowel evacuants, bronchial dilators, cardiovascular agents, CNS stimulants, chlators, cholesterol reducers, antihyperlipemics, contraceptives, cough and cold preparations, decongestants, expectorants, deodorants, dermatologicals, diagnostics, dietary supplements, diuretics, depamine receptor antagonists, electrolytes, emetics, enzymes, digestants, enzyme inhibitors, ergot derivatives, fertility agents, goluctolinetics, gall stone dissolvers, GI motility regulators, geriatric agents, hematinics, hemostatics, histamine H2 antagonists, antihemorrhoidals, hormones, hyperglycemic agents, hypnotics, immunosuppressives, antiinsect bite agents, laxatives, lipotropics, monoclonal antibodies, mouthwashes, mucolytics, muscle relaxants, narcotic antagonists, narcotic detoxifiers, nasal agents, opthalmologicals, otic agents, oxytocics, parasympatholytics, parasympathomimetics, penicillin adjuvants, plasma extenders, plasma fractions, prostaglandins, psychostimulants, respiratory stimulants, sclerotics, sedatives, antismoking agents, sympatholytics, sympathomimetics, thrombolytics, thyroid preparations, tranquilizers, antituberculars, uricosuries, urinary acidifiers, urinary tract agents, uterine contractants, uterine contraction inhibitors, vaginal preparations, vitamins and x-ray contrast media. The above terms are used in the manner common to the pharmaceutical sciences as exemplified by the Physicians Desk Reference, 42st edition (Medical Economics Co. NJ, 1988).

Lipid-soluble nutrients include edible oils from all sources (vegetable, fish, and the like), vitamins, carbohydrate derivatives, amino acid derivatives, protein derivatives, enzymes, fats, sterols, and all other lipid soluble food additives listed and generally recognized as safe (both currently and in the future since the invention is independent of the particular lipid-soluble material) in 21 CFR 170- 1999 and other parts of the Code of Federal Regulations. (U.S. Government Printing Office, Washington, DC, 1988)

The oil-in-water emulsifiers useful herein include surface active agents capable of forming oil-in-water emulsions and suitable for use orally or parenterally depending on the intended mode of administration. For oral products, examples include those emulsifiers currently listed in 21 CFR 170-199 and other parts of the Code of Federal Regulations which are generally recognized as safe in man or appropriate animals. For parenteral products, useful emulsifiers include natural and synthetic phospholipids, egg and soy lecithins, block copolymers of ethylene oxide and propylene oxide (Pluronics^{tm}, Tetronics^{tm} - BASF Corp.), straight chain POE derivatives (Tweens^{tm} - ICI America), sorbitan esters (Arlacels^{tm} - ICI America) or cholic acid derivatives.

Carbohydrates useful herein are those carbohydrates which are both hydrophilic and water-soluble. The carbohydrate is thus one which is capable of being dehydrated to a dry solid (so that the lipid-soluble materials have a place on which to deposit during the drying operation). The specific carbohydrate used should also be biocompatable with the intended use of the specific emulsion. Examples of such hydrophilic water-soluble carbohydrates include mono, di, oligo, and polysaccharides and sugar alcohols. More specifically, useful herein are dextrose, sucrose, fructose, galactose, sorbitol, mannitol, xylitol, trehalose, inositol, lactose, maltose, dextrin, and water-soluble starch derivatives are suitable carbohydrates. Any other hydropohilic, water-soluble carbohydrate which either now or in the future becomes generally recognized as safe for food use may be used herein.

While any suitable source of water may be used both in preparing the initial emulsions as well as in reconstituting the dehydrated ones, generally the purity of the water will depend upon the specific use intended for the product. The aqueous phase may also contain, but not necessarily dissolved therein, other components known to the art to be appropriate for the intended route of administration, e.g. water-soluble materials such as buffers, chelates, electrolytes, flavors, colors, essential elements, salts, amino acids, water soluble vitamins, preservatives, stablilizers and combinations thereof.

The precursor oil-in-water emulsion is conventionally prepared by (i) dissolving all lipid-soluble active materials, emulsifiers and additives into a lipid phase, (ii) dissolving all water-soluble or water-dispersible emulsifiers, carbohydrates, and additives into an aqueous phase, and (iii) forming an oil-in-water emulsion of the two phases wherein the oil phase droplets have average diameters of less than about 0.4 µm.

Heat may be used, where appropriate, to achieve dissolution for either or both of the phases, provided that none of the components is subject to rapid heat-degradation.

Critical to the successful rehydration of the subsequently dehydrated emulsion is the preparation of a precursor liquid emulsion with a submicron average droplet size (i.e. less than about 0.4 µm and also, preferably, a narrow size distribution. The average diameter of the droplets is measured by quasielastic laser light scattering instrumentation. Preferably, the average droplet size is less than 0.3 µm with a size distribution less than +/- 50%. Conventional mixing technology is seldom able to achieve such size and size distribution without employing unacceptably high levels of surface active agents or requiring the presence of undesirable cosolvents such as alcohols and the like. The preferable droplet size range may be readily achieved by employing a process exemplified by Microfluidizer^{R} technology. Microfluidizer equipment is commercially available from Microfluidics Corp., Newton, MA, and is described in U.S. Pat. No. 4,533,254, the subject matter of which is hereby incorporated by reference.

To utilize the Microfluidizer equipment and technology, a crude emulsion is prepared by slowly adding one phase to the other, preferably the lipid phase to the aqueous phase, with low energy mixing provided by such as a conventional propeller stirrer. The crude emulsion is then processed through the ultra high energy Microfluidizer equipment to produce a superfine emulsion of submicron size and within the preferable average droplet size range. The precursor liquid emulsion exhibits excellent storage stability with regard to retaining its initial average size and size distribution.

The precursor oil-in-water emulsions are formed into the dehydrated compositions of this invention by dehydrating the precursor liquid emulsion. The dehydration can be performed at ambient or elevated temperature with or without vacuum. The drying temperature is generally as high as possible provided that it is not so high that any component is degraded thereby. Examples of suitable dehydration methods include spray drying, tray drying, drum drying, oven drying, falling ball drying, fluid bed drying or flash drying. In a preferred embodiment, it is desireable to minimize the length of exposure of the precursor liquid emulsion to both the atmosphere and to heat due to the sensitive nature of many of the ingredients employed and to minimize, preferably prevent, any degradation or agglomeration of the resulting dehydrated emulsion. Moreover, the drying process should provide an economic incentive relative to more costly processes such as lyophilization and provide a dehydrated emulsion that rehydrates into a liquid product similar to the precursor liquid emulsion. It has been discovered that spray drying combines all the aforementioned desirable elements. Spray drying entails creating droplets of the liquid emulsion using a nozzle or cetrifugal atomizer within a large chamber into which heated air is flowed. As the droplets encounter the hot air, the water evaporates and the resulting dry powder may be isolated by a cyclone or bag collector. Residence times are normally extremely short in a spray dryer, thus minimizing any deleterious contact with heat and air. Generally the spray drying will be performed at exit temperatures less than about 100°C. The droplet size of the original lipid phase, and not the size of the atomized emulsion droplet, has been found to determine the size of the droplets in the resulting emulsion on reconstitution of the dehydrated emulsion.

After spray drying, the dehydrated emulsions may be subjected to further manipulations such as sieving, sterilization and packaging as are well known in the art.

The practice of this invention is illustrated by the following examples in which all parts and percents are by weight unless otherwise specified:

### EXAMPLE I

### A. Precursor Liquid Emulsion Preparation

In a suitable container, 7.2 gm of egg phospholipid (parenteral grade; Pfanstiehl, Waukegan, IL) is added to 200 ml of water for injection, USP (Baxter, Morton Grove, IL) with propeller mixing until the phospholid is fully hydrated (1 hr.). Stirring continues while 60.0 g of soybean oil, USP, superefined (Welch, Holme & Clarke, S. Plainfield, NJ) is added dropwise. In another container, 30.0 g of sucrose, USP (Mallinkrodt, St. Louis, MO) is dissolved in 300 ml of water for injection, USP. The sucrose solution is slowly added, with propellar mixing, to the coarse emulsion of soybean oil, water and phospholipid described above and the total combined volume is then adjusted to 600 ml with water for injection, USP. The resulting coarse emulsion (droplet size 2 to 25 µm) is then adjusted to pH = 7.4 with either hydrochloric acid solution (reagent grade) or sodium hydroxide solution (reagent grade).

This material is them processed through a Microfluidizer^{R} M-110 (Microfluidics Corp., Newton, MA) four times at 12,000 psi operating pressure and a flow rate of 200 ml/minute. The resulting droplet size of the microemulsion was measured using a quasi-elastic laser light scattering particle size determination instrument (Brookhaven Instruments, Model BI-90, Brookhaven, NY). The average droplet size was 0.19 µm with a size distribution of +/- 35%.

### B. Dehydration of Emulsion

The precursor liquid emulsion described in (A) was dehydrated by spray drying using a Buchi Laboratory Model 190 spray dryer (Brinkmann Instruments, Westbury, NY). Flow rate, inlet heating, pump and aspirator rates were adjusted to achieve an outlet air temperature of 43°C. Dry product was collected for examination and analysis. It was white-light tan in color and showed acceptable flow characteristics. Analysis indicated a residual moisture content of 1.0%.

### C. Reconstitution of Dehydrated Emulsion

The dehydrated emulsion of (B) was weighed into vials (0.21 g per vial) which were then sealed with rubber diaphrams and metal closures. Using a syringe, 1.3 ml of water for injection was added to several of the vials which were then shaken for five seconds by hand. A white, fluid homogeneous appearing emulsion resulted in each case. Analysis of the emulsions for average droplet diameter showed that they were 0.21 µm with a size distribution of +/- 34%. Reconstituted emulsions showed no separation or meaningful change in average droplet size after storage for one week at room temperature.

### EXAMPLE II

Precursor liquid emulsion was prepared as in Example I using an additional 30 g of sucrose. Droplet size measurement after Microfluidizer^{R} processing was 0.20 µm with a size distribution of +/- 30%. This emulsion was dehydrated under the same drying conditions as Example I and similarly rehydrated. The rehydrated emulsion showed an average droplet size of 0.22 µm with a size distribution of +/- 28%.

### EXAMPLE III

Precursor liquid emulsion of Example II was dehydrated by spray drying with conditions adjusted so as to achieve an outlet air temperature of 48°C. The dehydrated emulsion, rehydrated as in Example I, showed a droplet size of 0.23 µm with a size distribution of +/- 27%.

### EXAMPLE IV

Precursor liquid emulsion of Example II was dehydrated by spray drying with conditions adjusted so as to achieve an outlet air temperature of 59°C. The dehydrated emulsion, reconstituted as in Example I, showed a droplet size of 0.21 µm with a size distribution of +/- 29%.

### EXAMPLE V

The following precursor liquid emulsion was prepared as in Example I:
- Soybean Oil: 30.0 g
- Egg Phospholipid: 7.2 g
- Sucrose: 30.0 g
- Water for injection: qs. to 600 ml

The pH was adjusted to 7.4. A droplet size determination of this precursor liquid emulsion was 0.21 µm with a size distribution of +/- 29%. This emulsion was then dehydrated by spray drying with conditions adjusted so as to achieve an outlet air temperature of 45°C. the dehydrated emulsion, reconstituted as in Example I, showed a droplet size of 0.19 µm with a size distribution of +/- 43%.

### EXAMPLE VI

Precursor liquid emulsion was formulated and prepared as in Example V using an additional 30 g of sucrose. A droplet size determination on this precursor liquid emulsion showed a size of 0.18 µm with a size distribution of +/- 30%. The emulsion was dehydrated as in Example V. Upon rehydration, size measurement of the droplets showed 0. 18 µm with a size distribution of +/- 38%. After three months storage at room temperature, this sample measured 0.16 µm with a size distribution of +/- 47%.

### EXAMPLE VII

The following precursor emulsion was prepared as in Example I:
- Soybean Oil: 500 g
- Egg Phospholipid: 60 g
- Sucrose: 750 g
- Water: qs to 5000 ml

A droplet size determination was made of the oil phase droplets in this precursor liquid emulsion and they were found to average 0.18 µm with a size distribution of +/- 35%. This emulsion was then dehydrated by spray drying using an APV Anhydro Dryer-Type S1 using an inlet air temperature of 200°C. and an outlet air temperature of 90°C. The dehydrated emulsion, upon reconstitution, showed droplets having an average size of 0.26 µm +/- 45%.

### EXAMPLE VIII

The following precursor emulsion was prepared:
- Metronidazole: 3 g
- Soybean Oil: 60 g
- Egg Phospholipid: 7.2 g
- Sucrose: 90 g
- Water for injection: qs 600 ml

Metronidazole, a therapeutic agent, is dissolved in the soybean oil by means of a propeller mixer. Egg phospholipid is added to the water and fully hydrated (1 hr) using propeller mixing. Mixing is continued and the sucrose is added slowly until complete dissolution is achieved at which time the oil phase is added forming coarse emulsion. This emulsion is then processed through a Microfluidizer M-110 three times at 14,000 psi operating pressure. The resulting precursor liquid emulsion, which contains oil phase droplets measuring 0.19 µm with a size distribution of +/-26% was dehydrated using a Buchi Laboratory Model 190 spray dryer. Upon reconstitution, the resulting emulsion showed an average droplet size of 0.27 µm with a size distribution of +/- 43%.

### COMPARATIVE EXAMPLE A

The basic procedure of Example I was repeated except that the hydrophilic, water-soluble starch, i.e. sucrose, was replaced by the hydrated undextrinized lipophilic starch of U.S. Pat. No. 4,492,714, i.e. National Starch # 1817. The specific composition prepared contained the following components:
- Egg phospholipid: 1.2 g
- Soybean oil: 10.0 g
- National Starch #1817: 7.0 g
- Water for injection: qs 100 ml

The composition was processed through the Microfluidizer as in Example I. The resultant precursor emulsion was not sufficiently stable to be dehydrated since it separated into two distinct phases within an hour of its preparation. As such, it is not suitable to produce a homogeneous dehydrated product which can be rehydrated to an essentially identical product.

### COMPARATIVE EXAMPLE B

The basic procedure of Example I was repeated with a different composition which, on a solids basis, is within the scope of U.S. Pat. No. 4,492,714. The specific composition evaluated was:
- Egg phospholipid ("emulsifier"): 0.3 g
- Soybean oil (" fat"): 7.5 g
- National Starch #1817: 1.5 g
- Sucrose ("edible filler"): 0.4 g
- Water for injection: qs 100 ml

The composition was processed into an oil-in-water emulsion in which the average droplet size was found to be about 0.5 µm with a size distribution of +/- 50%. The emulsion was dehydrated by tray drying at a maximum temperature of 50° C., which is essentially the actual temperature of the corresponding material that exits the spray drier in Example I.

Attempts to reconstitute the dehydrated emulsion by the addition of water were unsuccessful. The resulting liquid separated into two phases immediately upon cessation of shaking.

## Claims

1. A solid dehydrated oil-in-water emulsion which is prepared from an initial completely liquid oil-in-water emulsion which contains a continuous completely liquid aqueous phase, dispersed discontinuous completely liquid oil phase droplets therein, and containing no insoluble solid particulate matter, said droplets having an average diameter of less than about 0.4 µm, wherein
(a) the initial oil-in-water emulsion is prepared in the absence of any organic solvent and from a combination consisting of:
(i) 0.1 to 60 weight percent of a lipid-soluble material,
(ii) 0.1 to 10 weight percent of an oil-in-water emulsifier,
(iii) 0.5 to 70 weight percent of a hydrophilic, water-soluble carbohydrate which is solid at room temperature, and
(iv) 20 to 99 weight percent water,
(b) the dehydration is performed at a temperature of at least 20°C.; and
(c) after addition of water to reconstitute the initial emulsion, a completely liquid oil-in-water emulsion containing no insoluble solid particulate matter and again having dispersed oil phase droplets therein having an average diameter of less than about 0.4 µm is produced.

2. The product of Claim 1 wherein the lipid-soluble material is a lipid.

3. The product of Claim 1 wherein the lipid-soluble material is a human or animal food product.

4. The product of Claim 1 wherein the lipid-soluble material is a glyceride selected from the group consisting of synthetic and semi-synthetic mono-, di-, tri-glycerides.

5. The product of Claim 1 wherein the lipid-soluble material is a lipid-soluble nutrient selected from the group consisting essentially of edible vegetable oils, edible fish oils, vitamins, carbohydrate derivatives, amino acid derivatives, protein derivatives, enzymes, fats, and sterols.

6. The product of Claim 1 wherein the lipid-soluble material is a combination of a lipid and at least one other lipid-soluble material.

7. The product of Claim 6 wherein the other lipid-soluble material is selected from the group consisting of a nutrients, therapeutics, and biologics.

8. The product of Claim 1 wherein the emulsifier is selected from the group consisting essentially of phospholipids, egg and soy lecithins, block copolymers of ethylene oxide and propylene oxide, straight chain polyoxyethylene derivatives, sorbitan esters, and cholic acid derivatives.

9. The product of Claim 1 wherein the emulsifier comprises a phospholipid-containing emulsifier derived from eggs or soybeans.

10. The product of Claim 1 wherein the carbohydrate is selected from the group consisting essentially of dextrose, sucrose, fructose, galactose, sorbitol, mannitol, xylitol, trehalose, inositol, lactose, maltose, dextrin; starch derivatives, and mixtures thereof.

11. The product of Claim 1 wherein the water further contains at least one water-soluble material selected from the group consisting essentially of buffers, chelates, electrolytes, flavors, colors, essential elements, salts, amino acids, water-soluble vitamins, preservatives, and stablilizers.

12. A method of preparing a solid oil-in-water emulsion product according to claim 1 from which the water is removed and which can be reconstituted by the addition of water to produce a completely liquid emulsion which contains no solid particulate matter and wherein the oil phase droplets are less than about 0.4 µm in average diameter which comprises the steps of (i) forming a completely liquid precursor oil-in-water emulsion in the absence of an organic solvent and insoluble solid particulate matter from a mixture consisting of a lipid-soluble material, an oil-in-water emulsifier, a hydrophilic water-soluble carbohydrate which is solid at room temperature, and water, (ii) processing the completely liquid precursor emulsion into a completely liquid microemulsion having oil phase droplets which have average diameters less than about 0.4 µm, and (iii) removing the water from the microemulsion at a temperature from above about 20°C. to a maximum temperature which is below that at which any component in the mixture will substantially degrade.

13. The method of Claim 12 wherein the water removal is performed by means of a spray dryer.

14. The method of Claim 13 wherein the spray dryer is operated at a maximum exit temperature of less than about 100°C.

## Patentansprüche

1. Eine feste dehydrisierte Öl-in-Wasser-Emulsion, die aus einer anfänglichen, vollständig flüssigen Öl-in-Wasser-Emulsion zubereitet wird, welche eine kontinuierliche vollständig flüssige wäßrige Phase enthält, wobei darin diskontinuierlich vollständig flüssige Ölphasentröpfchen verteilt sind, und keine unlösbare Feststoffpartikelmaterie enthält und die Tröpfchen einen mittleren Durchmesser von weniger als ungefähr 0,4 µm aufweisen, bei welcher
(a) die anfängliche Öl-in-Wasser-Emulsion bei Abwesenheit irgendwelcher organischer Lösungsmittel und aus einer Kombination zubereitet wird, die besteht aus:
(i) 0,1 bis 60 Gewichtsprozent eines lipidlöslichen Materials,
(ii) 0,1 bis 10 Gewichtsprozent eines Öl-in-Wasser-Emulgierers,
(iii) 0,5 bis 70 Gewichtsprozent eines hydrophilen, wasserlöslichen Kohlenhydrates, das bei Raumtemperatur fest ist, und
(iv) 20 bis 99 Gewichtsprozent Wasser,
(b) die Dehydrisierung bei einer Temperatur von mindestens 20°C durchgeführt wird; und
(c) nach dem Hinzufügen von Wasser zur Rekonstitution der anfänglichen Emulsion eine vollständig flüssige Öl-in-Wasser-Emulsion erzeugt wird, die keine unlösliche Feststoffpartikelmaterie enthält und die wieder darin verteilte Ölphasentröpfchen mit einem mittleren Durchmesser von weniger als ungefähr 0,4 µm aufweist.

2. Erzeugnis nach Anspruch 1, bei dem das lipidlösliche Material ein Lipid ist.

3. Erzeugnis nach Anspruch 1, bei dem das lipidlösliche Material ein menschliches oder tierisches Nahrungsmittelprodukt ist.

4. Erzeugnis nach Anspruch 1, bei dem das lipidlösliche Material ein Glycerid ist, das aus der Gruppe ausgewählt ist, die aus synthetischen oder halbsynthetischen Mono-, Di-, Triglyceriden besteht.

5. Erzeugnis nach Anspruch 1, bei dem das lipidlösliche Material ein lipidlöslicher Nährstoff ist, der aus der Gruppe ausgewählt ist, die im wesentlichen besteht aus eßbaren vegetarischen Ölen, eßbaren Fischölen, Vitaminen, Kohlenhydratderivaten, Aminosäurederivaten, Proteinderivaten, Enzymen, Fetten und Sterolen.

6. Erzeugnis nach Anspruch 1, bei dem das lipidlösliche Material eine Kombination aus einem Lipid und mindestens einem anderen lipidlöslichen Material ist.

7. Erzeugnis nach Anspruch 6, bei dem das andere lipidlösliche Material aus der Gruppe ausgewählt ist, die aus Nährstoffen, Therapeutika und biologischen Stoffen besteht.

8. Erzeugnis nach Anspruch 1, bei dem der Emulgierer aus der Gruppe ausgewählt ist, die im wesentlichen aus Phospholipiden Ei- und Sojalecithinen, Blockcopolymeren aus Äthylenoxyd und Propylenoxyd, Polyoxydäthylenderivaten mit gerader Kette, Sorbitanester und Cholinsäurederivaten besteht.

9. Erzeugnis nach Anspruch 1, bei dem der Emulgierer einen Phospholipid enthaltenden Emulgierer aufweist, der aus Eiern oder Sojabohnen stammt.

10. Erzeugnis nach Anspruch 1, bei dem das Kohlenhydrat aus der Gruppe ausgewählt ist, die im wesentlichen aus Dextrose, Saccharose, Fructose, Galactose, Sorbit, Mannit, Xylit, Trehalose, Inosit, Lactose, Maltose, Dextrin, Stärkederivaten und deren Mischungen besteht.

11. Erzeugnis nach Anspruch 1, bei dem das Wasser weiter mindestens ein wasserlösliches Material enthält, das aus der Gruppe ausgewählt ist, die im wesentlichen aus Pufferstoffen, Chelate, Elektrolyten, Aromastoffen, Farbstoffen, essentiellen Elementen, Salzen, Aminosäuren, wasserlöslichen Vitaminen, Konservierungsstoffen und Stabilisatoren besteht.

12. Verfahren zum Zubereiten eines festen Öl-in-Wasser-Emulsionserzeugnisses nach Anspruch 1, aus dem das Wasser entfernt wird und das durch Hinzufügen von Wasser rekonstituiert werden kann, um eine vollständig flüssige Emulsion zu erzeugen, die keine Feststoffpartikelmaterie enthält und bei der die Ölphasentröpfchen im mittleren Durchmesser kleiner als ungefähr 0,4µm sind und das die Schritte aufweist: (i) Bilden einer vollständig flüssigen Vorgänger-Öl-in-Wasser-Emulsion bei Abwesenheit eines organischen Lösungsmittels und unlösbarer Feststoffpartikelmaterie aus einer Mischung, die aus einem lipidlöslichen Material, einem Öl-in-Wasser-Emulgierer, einem hydrophilen wasserlöslichen Kohlenhydrat, das bei Raumtemperatur fest ist, und Wasser besteht, (ii) Verarbeiten der vollständig flüssigen Vorgängeremulsion in eine vollständig flüssige Mikroemulsion mit Ölphasentröpfchen, die einen mittleren Durchmesser von weniger als ungefähr 0,4 µm aufweisen, und (iii) Entfernen des Wassers aus der Mikroemulsion bei einer Temperatur von über ungefähr 20°C bis zu einer maximalen Temperatur, die unterhalb derjenigen liegt, bei der sich irgendeine Komponente in der Mischung im wesentlichen zersetzen wird.

13. Verfahren nach Anspruch 12, bei dem das Entfernen des Wassers mittels eines Sprühtrockners durchgeführt wird.

14. Verfahren nach Anspruch 13, bei dem der Sprühtrockner bei einer maximalen Ausgangstemperatur von weniger als ungefähr 100°C betrieben wird.

## Revendications

1. Emulsion huile-dans-eau déshydratée, solide, qui est préparée à partir d'une émulsion initiale huile-dans-eau totalement liquide qui contient une phase aqueuse liquide totalement continue, contenant des gouttelettes dispersées de phase huileuse discontinue et totalement liquide, et ne contenant pas de matière particulaire solide insoluble, le diamètre moyen desdites gouttelettes étant inférieur à environ 0,4 µm, dans laquelle
(a) l'émulsion initiale huile-dans-eau est préparée en l'absence de tout solvant organique et à partir d'une combinaison constituée de :
(i) 0,1 à 60 pour cent en poids d'une matière liposoluble ;
(ii) 0,1 à 10 pour cent en poids d'un émulsionnant huile-dans-eau ;
(iii) 0,5 à 70 pour cent en poids d'un glucide hydrophile, hydrosoluble, qui est solide à température ambiante, et
(iv) 20 à 99 pour cent en poids d'eau,
(b) la déshydratation est réalisée à une température d'au moins 20 °C ; et
(c) après l'addition d'eau pour reconstituer l'émulsion initiale, une émulsion huile-dans-eau complètement liquide, ne contenant pas de matière particulaire solide insoluble et contenant à nouveau des gouttelettes de phase huileuse dispersées, dont le diamètre moyen est inférieur à environ 0,4 µm, est produite.

2. Produit selon la revendication 1, dans lequel la matière liposoluble est un lipide.

3. Produit selon la revendication 1, dans lequel la matière liposoluble est un produit alimentaire pour l'homme ou l'animal.

4. Produit selon la revendication 1, dans lequel la matière liposoluble est un glycéride choisi dans le groupe constitué de mono-, di-, triglycérides synthétiques et semi-synthétiques.

5. Produit selon la revendication 1, dans lequel la matière liposoluble est un nutriment liposoluble choisi dans le groupe constitué essentiellement d'huiles végétales comestibles, d'huiles de poisson comestibles, de vitamines, de dérivés glucidiques, de dérivés d'acides aminés, de dérivés protéiques, d'enzymes, de graisses, et de stérols.

6. Produit selon la revendication 1, dans lequel la matière liposoluble est une combinaison d'un lipide et d'au moins une autre matière liposoluble.

7. Produit selon la revendication 6, dans lequel l'autre matière liposoluble est choisie dans le groupe constitué des nutriments, des produits thérapeutiques, et des produits biologiques.

8. Produit selon la revendication 1, dans lequel l'émulsionnant est choisi dans le groupe constitué essentiellement des phospholipides, des lécithines d'oeuf et de soja, des copolymères en bloc d'oxyde d'éthylène et d'oxyde de propylène, des dérivés de polyoxyéthylène à chaîne linéaire, des esters du sorbitanne, et des dérivés de l'acide cholique.

9. Produit selon la revendication 1, dans lequel l'émulsionnant comprend un émulsionnant contenant un phospholipide dérivé d'oeuf ou de soja.

10. Produit selon la revendication 1, dans lequel le glucide est choisi dans le groupe constitué essentiellement du dextrose, du saccharose, du fructose, du galactose, du sorbitol, du mannitol, du xylithol, du tréhalose, de l'inositol, du lactose, du maltose, de la dextrine, des dérivés d'amidon, et de leurs mélanges.

11. Produit selon la revendication 1, dans lequel l'eau contient en outre au moins une matière hydrosoluble choisie dans le groupe constitué essentiellement des tampons, chélates, électrolytes, arômes, colorants, éléments essentiels, sels, acides aminés, vitamines hydrosolubles, conservateurs et stabilisants.

12. Méthode de préparation d'un produit d'émulsion huile-dans-eau solide selon la revendication 1,dont on extrait l'eau et qui peut être reconstitué par l'addition d'eau pour produire une émulsion complètement liquide qui ne contient pas de matière particulaire solide et dans laquelle le diamètre moyen des gouttelettes de phase huileuse est inférieur à environ 0,4 µm, qui comprend les étapes (i) de formation d'une émulsion précurseur huile-dans-eau complètement liquide en l'absence de solvant organique et de matière particulaire solide insoluble, à partir d'un mélange constitué d'une matière liposoluble, d'un émulsionnant huile-dans-eau, d'un glucide hydrophile hydrosoluble qui est solide à température ambiante, et d'eau, (ii) de transformation de l'émulsion précurseur complètement liquide en une micro-émulsion complètement liquide dont les gouttelettes de phase huileuse ont un diamètre moyen inférieur à environ 0,4 µm, et (iii) d'extraction de l'eau de la micro-émulsion à partir d'une température supérieure à environ 20 °C jusqu'à une température maximale qui est inférieure à la température de dégradation importante de l'un quelconque des composants du mélange.

13. Méthode selon la revendication 12 dans laquelle l'extraction de l'eau est réalisée à l'aide d'un sécheur par pulvérisation.

14. Méthode selon la revendication 13 dans laquelle le sécheur par pulvérisation fonctionne à une température maximale en sortie, inférieure à environ 100 °C.
